**Europäisches Patentamt**

**European Patent Office**

**Office européen des brevets**

(19)

(11) Veröffentlichungsnummer: **0 239 098 B1**

(12) # EUROPÄISCHE PATENTSCHRIFT

(45) Veröffentlichungstag der Patentschrift: **20.02.91**

(51) Int. Cl.⁵: **A61N 2/02**

(21) Anmeldenummer: **87104421.0**

(22) Anmeldetag: **25.03.87**

(54) **Magnetfeldmatte.**

(30) Priorität: **27.03.86 DE 8608438 U**

(43) Veröffentlichungstag der Anmeldung:
**30.09.87 Patentblatt 87/40**

(45) Bekanntmachung des Hinweises auf die
Patenterteilung:
**20.02.91 Patentblatt 91/08**

(84) Benannte Vertragsstaaten:
**AT BE CH DE FR GB IT LI LU NL SE**

(56) Entgegenhaltungen:
**DE-A- 2 623 012**
**DE-A- 3 333 707**
**DE-B- 2 828 097**
**FR-A- 716 954**
**FR-A- 1 371 236**

(73) Patentinhaber: **Schmidt, Peter**
**Dörferstrasse 10**
**D-8940 Memmingen(DE)**

(72) Erfinder: **Schmidt, Peter**
**Dörferstrasse 10**
**D-8940 Memmingen(DE)**

(74) Vertreter: **Sperling, Rüdiger, Dipl.-Ing.**
**Patentanwälte Dipl.Ing.S. Staeger**
**Dipl.Ing.Dipl.Wirtsch.Ing. R Sperling Müller-**
**strasse 31**
**D-8000 München 5(DE)**

EP 0 239 098 B1

## Beschreibung

Die Erfindung betrifft eine Matte zur Verwendung als Liege-, Sitz- oder Lehnenmatte, mit einer von einer Hülle umgebenden Füllung aus mindestens einer aus Schaumgummi oder Schaumstoff bestehenden Polsterlage und einer Einlage aus Metall.

Aus der DE-AS 28 28 097 ist ein Möbel, z.B. Liege oder Sessel zum Entspannen des menschlichen Körpers bekannt, der einen elektromagetischen Generator mit einer Resonanzantenne und einer Betriebsfrequenz der elektromagnetischen Wellen im Band von 5 - 300, vorzugsweise 30 - 160 pulsierende Wellen pro Sekunde aufweist.

Aus der DE-OS 33 33 707 ist eine Untermatratze bekannt, bei der die Einlage aus einer Aluminiumfolie besteht. Diese Aluminiumfolie ist zwischen zwei Schaumstofflagen aufgenommen und soll insbesondere der Wärmeisolierung dienen.

Aus der DE-OS 31 41 668 ist eine als isolierende Unterlage dienende Matte bekannt, bei der auf einer Polyäthylenschaumschicht eine mit Aluminium bedampfte Polyäthylenfolie als oberste Schicht aufgebracht ist. Auch eine derartige Matte dient der Wärmeisolierung, wobei zur besseren Luftzirkulation die Aluminiumschicht porig ausgebildet ist.

Diese bekannten Matten wirken somit lediglich abschirmend gegen Wärme, besitzen aber ihrerseits keine aktive Einwirkmöglichkeit auf den Menschen.

Es ist bekannt, daß Wechselfelder und elektromagnetische Wellen einen wesentlichen Einfluß auf den menschlichen Körper haben können und Schwankungen beispielsweise der Luftelektrizität biologische Wirkungen auf Menschen haben können. In der Medizin werden die Wärmewirkung hochfrequenter Ströme zu Therapiezwecken ausgenutzt, wobei Forschungen gezeigt haben, daß Heilerfolge erzielt werden können. Die Indikationen zur Anwendung von Instrumenten, die auf der bezeichneten Erkenntnis beruhen, können typischen Serotoninreizesein. Es ist bekannt, daß mit folgenden Frequenzen Erfolge erzielt worden sind. Bei

1. Frequenz 2,5 Hz:akute Entzündungen.

2. Frequenz 5 - 7 Hz:leichte Reizzustände (Nervosität, Schlafstörungen, Hypertonie, Tachykardie, vegetative Dystonie).

3. Frequenz 10 - 14 Hz:schwere Reizzustände, Migräne, Seekrankheit, Reisekrankheit, Narbenschmerzen, Phantomschmerzen, Rheumaschmerzen, Arthrosen, allergische Reaktionen, Asthma.

Die Wirkung derartiger Magnetwechselfelder wird wie folgt erklärt: Bei Erkrankungen sind die bioelektrischen Vorgänge im Körper gestört. Sie laufen entweder zu rasch oder gehemmt ab. Diese bioelektrischen Vorgänge im Körper können entweder aktiviert oder gebremst werden, um sie wieder auf die übliche Norm zurückzuführen. Das Elektrowechselfeld erzeugt im Körper Wirbelströme, durch die sich die elektrischen Ladungen in den Zellmembranen verändern. Die so erzeugten Reize wirken sich normalisierend auf das vegetative Nervensystem aus und somit auch auf die Zielorgane. Beobachtungen haben gezeigt, daß abhängig von der Frequenz sich beispielsweise Blutgefäße erweitern oder verengen. Die Magnetfeldtheorie ist insbesondere auch in der klinischen Medizin zur Verbesserung der Heilung von Knochenbrüchen und Blutgefäßen bekannt.

Auch Behandlungserfolge bei psychosomatischen Beschwerden sind statistisch nachgewiesen, rheumatische Leiden konnten wesentlich verringert werden.

Die bekannten Geräte sind jedoch im wesentlichen ortsbezogene Geräte, und eine Therapie erfordert stets einen gewissen Zeit-bedarf, während welcher Zeit der Patient seiner üblichen Beschäftigung nicht weiter nachgehen kann.

Der Erfindung liegt daher die Aufgabe zugrunde, eine Vorrichtung zu schaffen, die es gestattet, die Magnetwechselfelder anzuwenden, wenn sich der Körper üblicherweise in Ruhe befindet.

Erfindungsgemäß wird bei einer Matte gattungsgemäßer Art vorgeschlagen, daß die Einlage eine zu einer mäander förmigen Magnetfeldspule angeordnete Drahtwicklung ist und die freien Enden des Spulendrahts mit dem Magnetfeld im pulsgeber verbunden sind. Die Spule Kann in oder unter einer Polsterhauptlage angeordnet sein. Diese Polsterhauptlage ist ein handelsüblicher Schaumstoff oder ein Schaumgummi. Insbesondere bietet sich als Material Latex an.

Der Spulendraht der Magnetfeldspule wird erfindungsgemäß mäanderförmig angeordnet, wodurch man ein relativ homogenes Magnetfeld über eine größere Fläche erhält. Günstig kann es sein, daß in einer Matte mehrere voneinander unabhängige Magnetfeldspulen zur Erzeugung örtlich unterschiedlicher Magnetfelder vorgesehen sind.

Dabei kann es vorteilhaft sein, daß der Spulendraht der unterschiedlichen Magnetfeldspulen verschiedene Dicken aufweist, um die unterschiedlichen Magnetfelder zu erzeugen.

Vorteilhaft ist es, wenn der Spulendraht aus mindestens 0,25 mm dickem ummaltem Kupferdraht besteht. Günstige Ergebnisse erzielt man dann, wenn in der Magnetfeldspule der jeweilige Abstand von einem Spulendraht zum anderen zwischen 5 cm und 20 cm beträgt, vorzugsweise 6cm.

Eine besonders große Anwendungskapazität wird dadurch erzielt, daß die freien Enden des Spulendrahts mit einem Magnetfeldimpulsgeber verbunden sind, welcher Magnetfeldimpulsgeber auf die gewünschte Frequenzzahl einstellbar ist.

Die Frequenz beträgt günstigerweise zwischen 2 bis 20 Hz.

Der Magnetfeldimpulsgeber weist üblicherweise einen Ein-Aus-Schalter auf, einen Testschalter mit Leuchtdiode, einen Drehregler zur Festlegung der Frequenz, sowie einen Ausgang für einen Klinkenstecker. Üblicherweise wird er mittels einer Batterie betrieben, Adapter für einen Wechselstromanschluß können jedoch auch vorgesehen sein.

Die Impulsbreite beträgt üblicherweise 50 Mikrosekunden, wobei der Stromverbrauch ohne LED bei 10 Hz ca. 0,05 mA mit LED, während des Test ca. 2 mA beträgt. Als Betterie wird eine 9V Block-Batterie verwendet, die am Ausgang eine Spannung von ca. 8 - 9 V erzeugt.

Bei einer vorteilhaften Ausbildung der Matte kann vorgesehen sein, daß die Schaumstoffhauptlage auf einer ihrer Seiten, als Schnittseite bezeichnet, mit in der gewünschten mäanderförmigen Spulform angeordneten Einschnitten versehen ist, in welchen der Spulendraht eingebettet ist. Durch diese Maßnahme ist es nicht erforderlich, besondere Befestigungsmaßnahmen, wie z.B. Aufkleben des Drahts auf der Schaumstoffhauptlage vorzusehen.

Günstig kann es sein, daß die Schnittseite der Schaumstoffhauptlage mit einer Decklage abgedeckt ist. Die Decklage wird dabei vorteilhafterweise zumindest an bestimmten Punkten an der Schnittseite mit der Schaumstoffhauptlage verklebt oder vernäht, so daß auch bei höherer Verschiebungs- und beispielsweise Faltbelastung die Lage der Spulendrähte stets fixiert ist und die Drähte beim Gebrauch nicht gespürt werden. Die neuerungsgemäße Matte kann sowohl als Untermatratze im Bett oder auch als direkte Liegematratze verwendet werden. Es sind auch kleine Ausführungsformen vorgesehen, bei denen die Matte lediglich die Größe eines Kopf- oder Sitzkissens aufweist, so daß die Therapie auch örtlich begrenzt durchführbar ist. Auch kann die erfindungsgemäße Matte in Sitzpolster oder Sitzüberzügen eingearbeitet sein, so daß beispielsweise eine gewünschte Therapie auch während einer Autofahrt vorgenommen werden kann - diesbezüglich wird darauf verwiesen, daß bei gezielter Anwendung die Aufmerksamkeit und das Aufnahmevermögen für das Verkehrsgeschehen erhöht werden kann.

Im folgenden wird die Erfindung anhand in der Zeichnung dargestellter Ausführungsbeispiele näher erläutert. Es zeigen:

Fig. 1     eine perspektivische Darstellung einer neuerungsgemäßen Matte zum Teil im Schnitt, zum Teil mit herausgebrochenen Bereichen,

Fig. 2     eine schematische Darstellung einer Magnetfeldspulenanordnung wie sie in der erfindungsgemäßen Matte Verwendung findet,

Fig. 3     eine schematische Darstellung verschiedener Magnetfeldspulenanordnungen in einer Matte,

Fig. 4     eine perspektivische schematische Darstellung eines Magnetfeldimpulsgebers, und

Fig. 5     eine schematische Darstellung eines Schaltkreises für den Magnetfeldimpulsgeber aus Fig. 4.

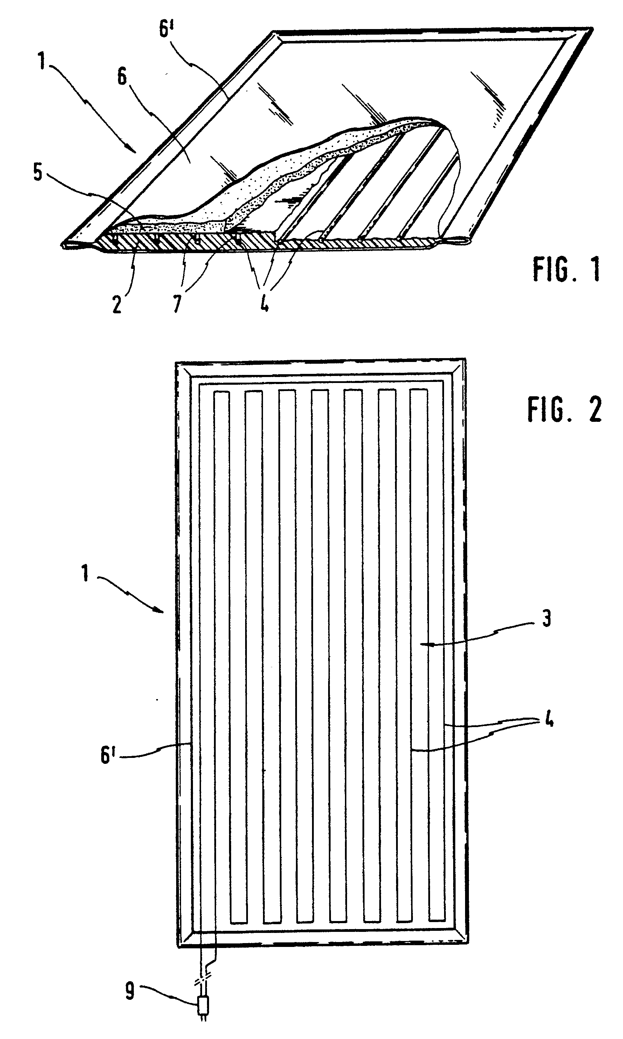

In Figur 1 ist eine perspektivische Darstellung einer Magnetfeldmatte 1 im Schnitt dargestellt. Die Matte 1 weist als Füllung eine Schaumstoffhauptlage 2 sowie eine Decklage 5 auf. Beide Lagen werden von einer Hülle oder einem Überzug 6 bedeckt, der imprägniert und schmutzabweisend sein kann. Der Überzug, die Schaumstoffhauptlage und die Decklage sind mittels einer am Rand entlanglaufenden Naht 6′ miteinander vernäht.

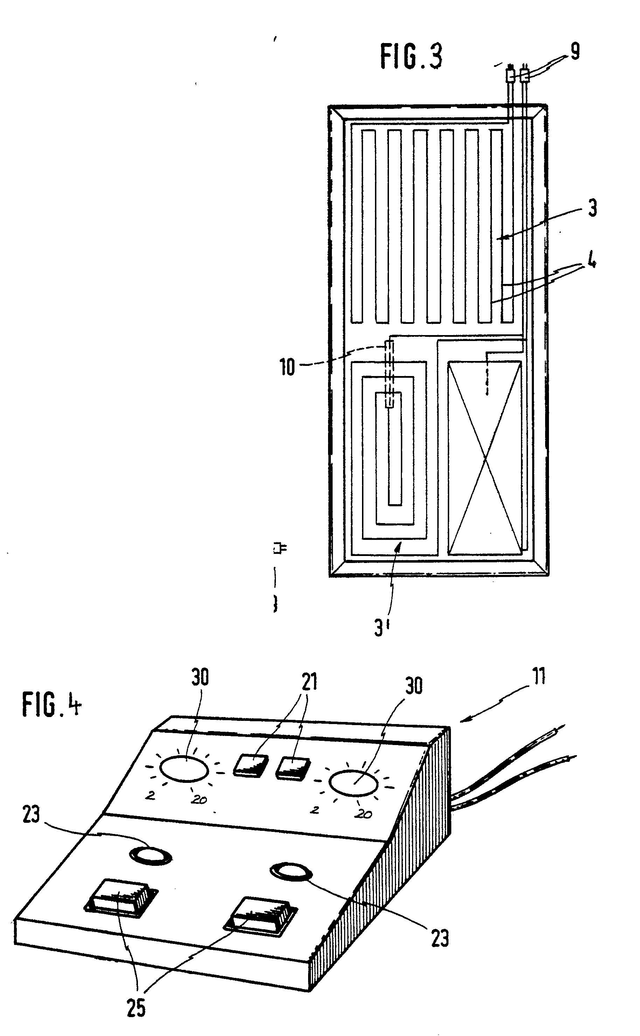

In der Schaumstoffhauptlage 2 sind Einschnitte 7 vorgesehen, deren geometrische Anordnung einer gewünschten Magnetfeldspulenanordnung, vergl. Figuren 2 und 3 entspricht.

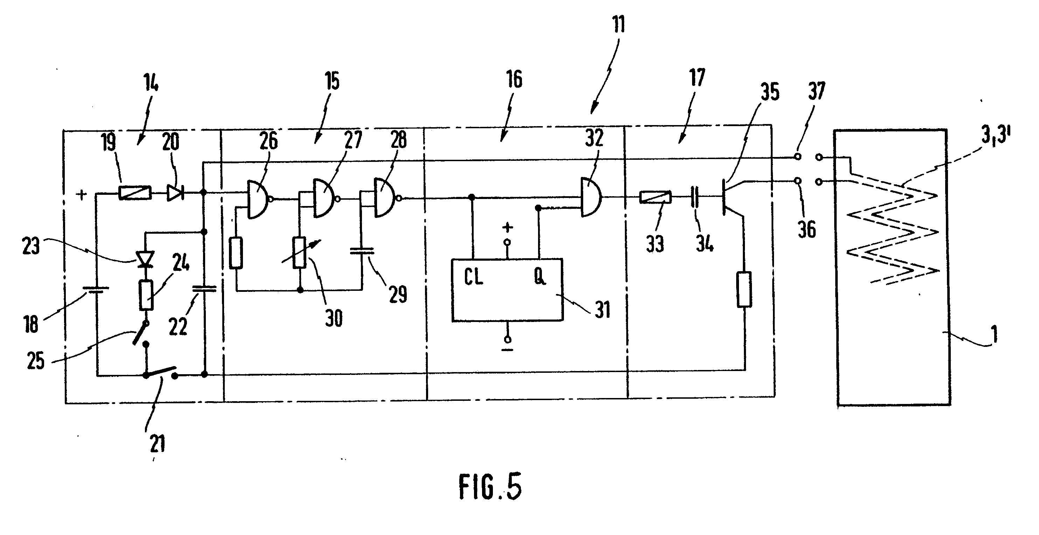

In den Einschnitten 7, deren Tiefe ca. der Hälfte der Dicke der Schaumstoffhauptlage entspricht, ist fortlaufend ein Spulendraht 4 eingelegt, so daß sich entsprechend dem Muster der Einschnitte eine Magnetfeldspulenkonfiguration ergibt, wie sie beispielsweise in den Figuren 2 und 3 dargestellt sind. In der in Fig. 2 dargestellten Ausführungsform ist der Spulendraht 4 mäanderförmig angeordnet, wobei die jeweiligen Enden des Spulendrahts in einem Stecker 9 zusammengefaßt sind, der in eine entsprechende Steckdose oder Buchse in einem Magnetfeld-Impulsgeber, (vergl. Fig. 4) vorgesehen ist.

Die Matte 1 kann so bemessen sein, daß sie als Normal- oder als Untermatratze in Betten eingelegt werden kann. Kleinere Abmessungen sind jedoch ebenfalls möglich, so beispielsweise in Form eines Sitzkissens, einer Rückenlehne oder Ausbildungen, die als Überzüge für Autositze, oder entsprechend angepaßt z.B. in Rollstühlen verwendet werden können.

Das in Fig. 3 dargestellte Ausführungsbeispiel zeigt eine Kombination verschiedener Magnetfeldspulenanordnungen bei einer Matte. So ist in dem dargestellten Beispiel im oberen Bereich die Magnetfeldspule 3 mäanderförmig ausgebildet, während im unteren Bereich zwei Magnetfeldspulen 3′ spiralenförmiger Konfiguration nebeneinander angeordnet sind. Auch bei dieser Ausbildung ist das innere Ende gegenüber der Magnetfeldspule abgeschirmt. Die genaue Ausbildung der Spirale kann von der dargestellten Konfiguration abweichen, beispielsweise ist es möglich, die Spiralform, nicht wie dargestellt, eckig auszubilden, sondern oval oder stetig gleichmäßig gekrümmt.

Auch ist die Stelle, an der die Spirale innen endet, bzw. außen beginnt, durch das dargestellte Beispiel nicht festgelegt, es kann jeweils die günstigste Form ausgewählt werden. Der Spulendraht weist üblicherweise eine Dicke von mindestens 0,25 mm auf und besteht aus einem ummantelten Kupferdraht. Es ist jedoch möglich, den Spulendraht 4 je nach Art der Magnetfeldspule 3 oder 3' mit einer unterschiedlichen Dicke auszustatten. Die Anordnung des Spulendrahts 4 zu einer Magnetfeldspule 3 oder 3' in der dargestellten Konfiguration geschieht üblicherweise in der Art, daß der Abstand von einem Spulendraht 4 zum anderen etwa 6 cm beträgt. Bei besonderen Anwendungsgebieten kann der Abstand geringer sein, er kann auch größer sein.

Durch den hohen Reibwert zwischen dem Material der Schaumstoffhauptlage und der Ummantelung des Kupferdrahts ist eine sichere Arretierung des Drahtes in den Einschnitten gewährleistet. Um jedoch auch bei hoher Belastung ein Austreten des Spulendrahts 4 aus den Einschnitten 7 zu verhindern, ist auf der Schnittseite 8 der Schaumstoffhauptlage 2 eine Decklage 5 angeordnet, die mit der Schaumstoffhauptlage 2 zumindest an bestimmten Stellen fest verbunden ist. Diese Verbindung kann durch Vernähen, beispielsweise durch Steppunkte oder Steppnähte oder auch durch ein doppelseitiges Klebeband bewerkstelligt werden.

In Figur 4 ist schematische in perspektivischer Darstellung ein Magnetfeldimpulsgeber 11 dargestellt, dessen Schaltkreis in Figur 5 schematisch dargelegt ist. Mit dem Impulsgeber 11 können kurze Impulse mit wählbarer Frequenz zwischen 2 und 20 Hz in die Spule 3,3' abgegeben werden, die in der Matte 1 eingebettet ist. Der Impulsgeber besteht im einzelnen aus stichpunktiert dargestellten Blöcken, nämlich einer Spannungsquelle 14, einem Oszillator 15 mit wählbarer Frequenz, einem Frequenzuntersetzer 16 und einer Leistungsendstufe 17.

Die Spannungsquelle 14 weist eine kleine Batterie 18 auf, deren Pluspol mit einem Widerstand 19 und einer Schutzdiode 20 verbunden ist, während mit dem Minuspol ein EIN/AUS-Schalter 21 verbunden ist. Zur Glättung des Batteriestromes ist noch ein zwischen dem Ausgang der Diode 20 und dem Schalter 21 parallel geschalteter Kondensator 22 vorgesehen.

In der Spannungsquelle 14 ist noch eine Testschaltung vorgesehen, die eine Serienschaltung aus einer Leuchtdiode 23, einem Widerstand 24 und einem Schalter 25 ist und zwischen dem positiven Ausgang der Spannungsquelle und dem Minuspol der Betterie 18 geschaltet ist. Wird der Schalter 25 geschlossen, so leuchtet die Leuchtdiode 23 auf, sofern die Batterie 18 noch ausreichende Spannung hat. Der Schalter 25 ist vorzugsweise ein Druckschalter.

Der positive Ausgang der Spannungsquelle 14 ist mit dem frquenzvariablen Oszillator 15 verbunden, der handelsüblicher Bauart ist und in der Regel aus mehreren NAND-Gliedern besteht, in diesem Falle aus drei NAND-Gliedern 26, 27 und 28, wobei der Ausgang des zweiten NAND-Gliedes über ein RC-Glied aus einem Kondensator 29 und einem Potentiometer 30 auf den zweiten Eingang des ersten NAND-Gliedes 26 rückgekoppelt ist. Das Potentiometer 30 dient zur Frequenzeinstellung, so daß am Ausgang des letzten NAND-Gliedes eine variable hohe Frequenz erscheint. Der Ausgang dieses NAND-Gliedes ist einem Takteingang CL eines Zählers 31 und dem ersten Eingang eines UND-Gliedes 32 zugeführt. Der Zähler 31 ist z.B. ein Ringzähler mit einer vorgegebenen Anzahl von Zählstufen, dessen Signalausgang Q einen Überlaufimpuls an den zweiten Eingang des UND-Gliedes 32 gibt, sobald die Anzahl der von dem Oszillator 15 abgegebenen Impulse der Anzahl der Zählstufen entspricht. In diesem Falle öffnet das UND-Tor 32 und überträgt einen Impuls über eine Koppelschaltung aus einem Widerstand 33 und einem Kondensator 34 an die Basis eines Leistungstransistors 35, der in Emitterschaltung betrieben wird. Der Kollektor des Transistors ist mit einem Ausgangsanschluß 36, der Pluspol der Batterie mit einem weiteren Anschluß 37 versehen, die zu einem Stecker zusammengefaßt sind. In diesen Stecker wird dann ein korrespondierende Stecker de Spule 3,3' eingesteckt.

Die mit einer solchen Schaltung in die Spule 3, 3' eingegebenen Impulse werden z.B. auf eine Impulsdauer von 50 µsec eingestellt; die Pulsbreite ist von dem Wert des Kondensators 29 abhängig. Der Stromverbrauch des Impulsgebers ist sehr gering und beträgt bei 10 Hz ca. 0,1 mA, so daß eine 9-Volt-Blockbatterie die Spule 3,3' zwei oder drei Monate versorgen kann.

Der Stromverbrauch des Impulsgebers im Testbetrieb, d.h. bei geschlossenem Schalter 25, ist etwa 4 mA.

Die Spulen weisen vorzugsweise einen ummantelten Kupferdraht mit einem Durchmesser von 0,25 mm auf; die Wicklungen der Spulen sind etwa im Abstand von 6 cm gelegt.

Mit dem beschriebenen Impulsgeber wurde ausgehend von einer magnetischen Induktion von 100 % in einem Abstand von 5 cm von der Spulenebene in einem Abstand von 10 cm noch 65% der magnetischen Induktion gemessen; im Abstand von 15 cm, 20 cm bzw., 25 cm noch 50 %, 33 % bzw. 20 % der magnetischen Induktion im Abstand von 5 cm.

Bei Verwendung einer anderen Spannungsquelle sind entsprechende elektrische Bauteile vorgesehen, so kann z.B. die Matte im Kraftfahrzeug mit der dort eingebauten Batterie betrieben werden, wenn entsprechende spannungsreduzierende Widerstände vorgeschaltet werden.

## Ansprüche

1. Matte zur Verwendung als Liege-, Sitz- oder Lehnenmatte, mit einer von einer Hülle umgebenen Füllung aus mindestens einer aus Schaumgummi oder Schaumstoff bestehenden Polsterlage, mit einem Magnetfeldimpulsgeber, der mit einer Frequenz zwischen 2 und 20 Hz arbeitet und mit einer zu mindestens einer Magnetfeldspule angeordneten Drahtwicklung, dadurch **gekennzeichnet**, daß der Spulendraht (4) der Magnetfeldspule (3,) mäanderförmig angeordnet ist und die freien Enden des Spulendrahts mit dem Magnetfeldimpulsgeber verbunden sind.

2. Matte nach Anspruch 1, dadurch **gekennzeichnet**, daß in der Matte (1) mehrere voneinander unabhängige Magnetfeldspulen (3,3') zur Erzeugung örtlich unterschiedlicher Magnetfelder vorgesehen sind.

3. Matte nach Anspruch 2, dadurch **gekennzeichnet**, daß der Spulendraht (4) der unterschiedlichen Magnetfeldspulen (3,3') verschiedene Dicken aufweist.

4. Matte nach mindestens einem der Ansprüch 1 bis 3, dadurch **gekennzeichnet**, daß der Spulendraht (4) aus mindestens 0,25 mm dickem ummanteltem Kupferdraht besteht.

5. Matte nach mindestens einem der Ansprüche 1 bis 4, dadurch **gekennzeichnet**, daß in der Magnetfeldspule (3,3') der jeweilige Abstand von einem Spulendraht (4) zum anderen zwischen 5 cm und 25 cm beträgt, vorzugsweise 6 cm.

6. Matte nach mindestens einem der Ansprüche 1 bis 5, dadurch **gekennzeichnet**, daß die Polsterhauptlage (2) auf einer ihrer Seiten, als Schnitteseite (8) bezeichnet, mit in der gewünschten Spulenform angeordneten Einschnitten (7) versehen ist, in welchen der Spulendraht (4) eingebettet ist.

7. Matte nach Anspruch 6, dadurch **gekennzeichnet**, daß die Schnittseite (8) der Polsterhauptlage (2) mit einer Decklage (5) abgedeckt ist.

8. Matte nach Anspruch 7, dadurch **gekennzeichnet**, daß die Decklage (5) zumindest an bestimmten Punkten an der Schnittseite (8) mit der Polsterhauptlage (2) verklebt oder vernäht ist.

## Claims

1. Matting for use in lying, sitting or leaning, having a filling, surrounded by a cover, the filling made of at least one upholstered layer consisting of foamed rubber material, and having a magnetic field pulse transmitter which operates at a frequency between 2 and 20 Hz and having winding wire arranged to form at least one magnetic field coil, characterised in that the coil wire (4) of the magnetic field coil (3) is arranged in a meander shape and the free ends of the coil wire are connected to the magnetic field pulse transmitter.

2. Matting according to Claim 1, characterised in that a plurality of magnetic field coils (3,3') independent of one another are provided in the matting(s) for generating locally different magnetic fields.

3. Matting according to Claim 2, characterised in that the coil wire (4) of the different magnetic field coils (3,3') is of different thicknesses.

4. Matting according to at least one of Claims 1 to 3, characterised in that the coil wire (4) consists of sheathed copper wire at least 0.25 mm thick.

5. Matting according to at least one of Claims 1 to 4, characterised in that in the magnetic field coil (3,3') the respective distance from one coil wire (4) to the other is between 5 cm and 25 cm, preferably 6 cm.

6. Matting according to at least one of Claims 1 to 5, characterised in that the upholstered main layer (2) is provided on one of its sides, designated as a cut side (8), with incisions (7) arranged in the desired coil form, in which incisions the coil wire (4) is embedded.

7. Matting according to Claim 6, characterised in that the cut side (8) of the upholstered main layer (2) is covered with a covering layer (5).

8. Matting according to Claim 7, characterised in that the covering layer (5) is bonded or sewn to the upholstered main layer (2) at least at points on the cut side (8).

## Revendications

1. Natte à utiliser comme natte de lit, de siège ou de dossier comportant un rembourrage entouré par une enveloppe, rembourrage constitué a'au moins une couche en caoutchouc mousse ou en produit alvéolaire, un impulseur de champ magnétique, qui travaille dans une fréquence comprise entre 2 et 20 Hz et un enroulement en fils disposé en au minimum un bobinage de champ magnétique, caractérisée en ce que le fil de bobinage (4) du bobinage (3) de champ magnétique a la forme de méandres et que les extrémités libres du fil de bobinage sont reliées à l'impulseur de champ magnétique.

2. Natte selon la revendication 1, caractérisée en ce que dans la natte (1) plusieurs bobinages (3,3') de champ magnétique, indépendants les uns des autres, sont prévus en vue de générer localement des champs magnétiques différents.

3. Natte selon la revendication 2, caractérisée en ce que le fil de bobinage (4) des différents bobinages de champ magnétique (3,3') présente des épaisseurs différentes.

4. Natte selon l'une au moins des revendications 1 à 3, caractérisée en ce que le fil de bobinage (4) est un fil de cuivre gainé d'au moins 0,25 mm d'épaisseur.

5. Natte selon l'une au minimum des revendications 1 à 4, caractérisée en ce que dans le bobinage de champ magnétique (3,3') la distance respective entre deux fils de bobinage (4) va de 5 à 25 cm, s'élevant de préférence à 6 cm.

6. Natte selon l'une au moins des revendications 1 à 5, caractérisée en ce que la couche principale de rembourrage(2) est pourvue sur une de ses faces, qualifiée de face découpée (8) d'entailles (7) placées selon la forme de bobinage souhaitée, entailles dans lesquelles le fil de bobinage (4) est encastré.

7. Natte selon la revendication 6, caractérisée en ce que la face découpée (8) de la couche principale de rembourrage (2) est recouverte d'une couche extérieure (5).

8. Natte selon la revendication 7, caractérisée en ce que la couche extérieure (5) est collée ou cousue à la couche principale de rembourrage (2) au minimum en des points déterminés de la face découpée (8).

FIG. 1

FIG. 2

7

FIG.3

FIG.4

FIG.5